# FASCICULE DE BREVET EUROPEEN

(11) **EP 1 851 301 B1**
(45) Date de publication et mention de la délivrance du brevet: **25.03.2015**
(21) Numéro de dépôt: 06709241.1
(22) Date de dépôt: 03.02.2006
(51) Int. Cl.: C11D 7/50, C08J 9/14, C07C 19/08, C07C 21/073

(54) **COMPOSITION A BASE DE TRANS-1,2 DICHLOROETHYLENE**
TRANS-1,2-DICHLOROETHYLEN-ZUSAMMENSETZUNG
TRANS-1,2 DICHLOROETHYLENE COMPOSITION

(30) Priorité: 23.02.2005 FR 0501832
(43) Date de publication de la demande: 07.11.2007
(73) Titulaire: ARKEMA FRANCE, 92700 Colombes (FR)
(72) Inventeur: LATIL, Laurent, F-38440 Moidieu de Tourbes (FR); ENAUX, Vincent, F-69690 Bessenay (FR)
(86) Numéro de dépôt international: PCT/FR2006/000252
(87) Numéro de publication internationale: WO 2006/090042

(56) Documents cités:
- EP-A- 1 435 371
- US-A- 3 349 039
- US-A- 6 100 229

## Description

La présente invention concerne une composition à base de trans-1,2 dichloroéthylène. Elle a plus particulièrement pour objet une composition non inflammable comprenant du trans-1,2 dichloroéthylène et d'au moins deux hydrofluorocarbures et ses utilisations.

Le trans-1,2 dichloroéthylène est un solvant chloré dont le point d'ébullition est de 48°C et qui au même titre que le trichloroéthane, le trichloroéthylène et le perchloroéthylène possède un bon pouvoir de solubilisation en particulier des corps gras (lubrifiants, huiles, graisses). Son usage a, jusqu'à présent, été relativement limité en raison de l'existence pour ce solvant d'un point d'éclair. Le trans-1,2 dichloroéthylène a effectivement un point éclair compris entre -11°C et -4°C dans les conditions standard de détermination (norme D3828-02 : coupe fermée, SETAFLASH).

Le brevet US 3,349,039 décrit des compositions à base de trans-1,2 dichloroéthylène et de trifluoro-1,1,2-trichloro-1,2,2-éthane ou de chlorure de méthylène, ces deux derniers composés permettant de supprimer le point éclair du trans-1,2 dichloroéthylène. Cependant, ces mélanges ne présentent guère d'intérêt maintenant car le trifluoro-1,1,2-trichloro-1,2,2-éthane est interdit depuis le protocole de Montréal et le chlorure de méthylène sévèrement réglementé (substance nocive, Cancérigène Mutagène Reprotoxique).

Le brevet US 6,100,229 décrit des compositions à base de mélanges azéotropiques trans-1,2 dichloroéthylène et 1,1,1,3,3-pentafluoropropane mais avec une forte teneur en 1,1,1,3,3-pentafluoropropane. L'inconvénient d'un tel mélange est son point d'ébullition relativement bas, valeur voisine de 20°C pour une composition contenant seulement 20 % de trans-1,2 dichloroéthylène.

Le document EP1435371 concerne des compositions de mousse contenant du trans-1,2-dichloroéthylène et un ou plusieurs hydrofluorocarbures, dont le 1,1,1,3,3-pentafluoropropane, le 1,1,1,3,3-pentafluorobutane, et le 1,1,1,2-tetrafluoroethane.

Par ailleurs, l'emploi du trans-1,2 dichloroéthylène avec un agent d'expansion dans la fabrication des mousses de polymères thermodurcissables est connu.

Dans beaucoup d'applications, les composants des mousses polyuréthane sont des prémélanges. Plus généralement, la formulation des mousses est prémélangée en deux composants. Le premier composant, plus connu sous la dénomination « composant A » comprend la composition isocyanate ou polyisocyanate. Le deuxième composant, plus connu sous la dénomination « composant B » comprend le polyol ou le mélange de polyols, l'agent tensioactif, le ou les catalyseurs, et le ou les agents d'expansion.

Le « composant B » pose des problèmes d'inflammabilité, même lorsque l'agent d'expansion entrant dans la composition du prémélange est non inflammable.

En outre, les problèmes d'élévation de pression des conteneurs renfermant le « composant B » sont souvent rencontrés lors de leur stockage.

La présente invention fournit donc des compositions qui permettent de résoudre tout ou en partie les problèmes précités.

La présente invention a donc pour premier objet une composition comprenant de 45 à 95 % en poids de trans-1,2 dichloroéthylène, de 1 à 45 % en poids de 1,1,1,3,3 pentafluoropropane et de 1 à 30 % en poids de 1,1,1,2 tetrafluoroethane (134a).

Egalement préférée est une composition comprenant de 80 à 95 % en poids de trains-1,2 dichloroéthylène, de 1 à 19 % en poids de 1,1,1,3,3 pentafluoropropane et de 1 à 15 % en poids de 1,1,1,2 tetrafluoroethane (134a).

La composition selon l'invention peut comprendre en outre un polyol ou un mélange de polyols.

La présente invention a donc pour deuxième objet une composition comprenant un polyol ou un mélange de polyols et le mélange ternaire de trans-1,2 dichloroéthylène, de 1,1,1,3,3 pentafluoropropane et de 1,1,1,2 tetrafluoroethane (134a) du premier objet.

Le mélange ternaire du premier objet représente de préférence entre 1 et 60 parties en poids pour 100 parties en poids de polyol ou mélange de polyols dans la composition du deuxième objet. Avantageusement, il représente entre 5 et 35 parties en poids pour 100 parties en poids de polyol ou mélange de polyols.

Comme polyols, on peut citer notamment le glycérol, l'éthylène glycol, le triméthylolpropane, le pentaérythritol, les polyétherpolyols, par exemple ceux obtenus par condensation d'un oxyde d'alkylène ou d'un mélange d'oxydes d'alkylène avec le glycérol, l'éthylène glycol, le triméthylolpropane, le pentaérythritol, les polyesterspolyols, par exemple ceux obtenus d'acides polycarboxyliques, notamment l'acide oxalique, l'acide malonique, l'acide succinique, l'acide adipique, l'acide maléïque, l'acide fumarique, l'acide isophtalique, l'acide téréphtalique, avec le glycérol, l'éthylène glycol, le triméthylolpropane, le pentaérythritol.

Les polyétherpolyols obtenus par addition d'oxydes d'alkylènes, en particulier l'oxyde d'éthylène et/ou l'oxyde de propylène, sur les amines aromatiques en particulier le mélange de 2,4 et 2,6 de toluène diamine conviennent également.

La présente invention a également pour objet un procédé de fabrication de mousses de polyuréthane. Ce procédé consiste à faire réagir un polyisocyanate organique (incluant le diisocyanate) avec la composition selon le deuxième objet. Cette réaction peut être activée à l'aide d'une amine et/ou d'autres catalyseurs et des agents tensio-actifs.

Comme polyisocyanate, on peut citer notamment les polyisocyanates aliphatiques avec un groupement hydrocarboné pouvant aller jusqu'à 18 atomes de carbone, les polyisocyanates cycloaliphatiques avec un groupement hydrocarboné pouvant aller jusqu'à 15 atomes de carbone, les polyisocyanates aromatiques avec un groupement hydrocarboné aromatique ayant de 6 à 15 atomes de carbone et les polyisocyanates arylaliphatiques avec un groupement hydrocarboné arylaliphatique ayant de 8 à 15 atomes de carbone.

Les polyisocyanates préférés sont le diisocyanato-2,4 et 2,6 toluyle, le diisocyanate de diphénylméthane, l'isocyanate de polyméthylènepolyphényle et leur mélange. Les polyisocyanates modifiés, tels que ceux contenant des groupements carbodiimides, des groupements uréthanes, des groupements isocyanurates, des groupements urée ou des groupements biurée peuvent également convenir.

La composition selon le premier objet peut être utilisée comme solvant. Les différentes applications sont notamment le traitement de surfaces solides, comme par exemple le nettoyage, le dégraissage, le séchage de surfaces solides et le défluxage de circuits imprimés, le nettoyage à sec des textiles, le nettoyage d'installations frigorifiques.

La composition selon le premier objet peut également être utilisée comme agent d'expansion des mousses de polymères thermodurcissables, par exemple les condensats de phénol/formol ou le polyuréthane. Elle convient tout particulièrement à la fabrication de mousses de polyuréthane.

La composition selon le premier objet peut également être utilisée comme fluides caloporteurs, agents de dépôt des silicones et/ou agents propulseurs d'aérosols.

### PARTIE EXPERIMENTALE

### EXEMPLE 1

On prépare une composition constituée de 100 g d'un polyol STEPANPOL PS2412 (type polyester) et de 7 g d'un mélange contenant 82% en poids de trans-1,2 dichloroéthylène, 15% en poids de 1,1,1,3,3-pentafluoropropane et 3% en poids de 1,1,1,2 tetrafluoroethane (134a). Cette composition ne présente pas de point éclair même à 80°C dans les conditions standard de détermination avec le test SETAFLASH, norme D3828-02.

### EXEMPLE 2

On prépare une composition constituée de 100 g d'un polyol STEPANPOL PS2412 (type polyester) et de 7 g d'un mélange contenant 85% en poids de trans-1,2 dichloroéthylène, 10% en poids de 1,1,1,3,3-pentafluoropropane et 5% en poids de 1,1,1,2 tetrafluoroethane (134a). Cette composition ne présente pas de point éclair même à 80°C dans les conditions standard de détermination avec le test SETAFLASH, norme D3828-02.

## Revendications

1. Composition comprenant de 45 à 95 % en poids de trans-1,2 dichloroéthylène, de 1 à 45 % en poids de 1,1,1,3,3 pentafluoropropane et de 1 à 30 % en poids de 1,1,1,2 tetrafluoroethane (134a).

2. Composition la revendication 1 comprenant de 80 à 95 % en poids de trans-1,2 dichloroéthylène, de 1 à 19 % en poids de 1,1,1,3,3 pentafluoropropane et de 1 à 15 % en poids de 1,1,1,2 tetrafluoroethane (134a).

3. Composition selon l'une quelconque des revendications précédentes comprenant en outre un polyol ou mélange de polyols.

4. Agent d'expansion comprenant une composition selon l'une quelconque des revendications précédentes.

5. Procédé de fabrication de mousses de polymères thermoplastiques en utilisant l'agent d'expansion selon la revendication 4.

6. Solvant comprenant une composition selon l'une quelconque des revendications 1 à 2.

## Patentansprüche

1. Zusammensetzung, umfassend 45 bis 95 Gew.-% trans-1,2-Dichlorethylen, 1 bis 45 Gew.-% 1,1,1,3,3-Pentafluorpropan und 1 bis 30 Gew.-% 1,1,1,2-Tetrafluorethan (134a).

2. Zusammensetzung nach Anspruch 1, umfassend 80 bis 95 Gew.-% trans-1,2-Dichlorethylen, 1 bis 19 Gew.-% 1,1,1,3,3-Pentafluorpropan und 1 bis 15 Gew.-% 1,1,1,2-Tetrafluorethan (134a).

3. Zusammensetzung nach einem der vorhergehenden Ansprüche, die außerdem ein Polyol oder Polyol-gemisch umfasst.

4. Treibmittel, umfassend eine Zusammensetzung nach einem der vorhergehenden Ansprüche.

5. Verfahren zur Herstellung von thermoplastischen Polymerschaumstoffen unter Verwendung des Treibmittels nach Anspruch 4.

6. Lösungsmittel, umfassend eine Zusammensetzung nach einem der Ansprüche 1 bis 2.

## Claims

1. Composition comprising from 45 to 95% by weight of trans-1,2-dichloroethylene, from 1 to 45% by weight of 1,1,1,3,3-pentafluoropropane and from 1 to 30% by weight of 1,1,1,2-tetrafluoroethane (134a).

2. Composition according to Claim 1, comprising from 80 to 95% by weight of trans-1,2-dichloroethylene, from 1 to 19% by weight of 1,1,1,3,3-pentafluoropropane and from 1 to 15% by weight of 1,1,1,2-tetrafluoroethane (134a).

3. Composition according to any one of the preceding claims, additionally comprising a polyol or a mixture of polyols.

4. Blowing agent comprising a composition according to any one of the preceding claims.

5. Process for the manufacture of thermoplastic polymer foams using the blowing agent according to Claim 4.

6. Solvent comprising a composition according to any one of Claims 1 to 2.
